# FASCICULE DE BREVET EUROPEEN

(11) **EP 2 765 960 B1**
(45) Date de publication et mention de la délivrance du brevet: **06.04.2016**
(21) Numéro de dépôt: 12770171.2
(22) Date de dépôt: 12.10.2012
(51) Int. Cl.: A61F 5/00

(54) **BANDE-ANNEAU GASTRIQUE GONFLABLE ET AJUSTABLE POUR LE TRAITEMENT DE L'OBESITE**
AUFBLASBARES UND EINSTELLBARES MAGENBAND ZUR BEHANDLUNG VON ADIPOSITAS
INFLATABLE AND ADJUSTABLE GASTRIC BAND FOR TREATING OBESITY

(30) Priorité: 12.10.2011 FR 1159237
(43) Date de publication de la demande: 20.08.2014
(73) Titulaire: Medical Innovation Developpement, 69570 Dardilly (FR)
(72) Inventeur: FRERING, Vincent, F-69660 Collonges-au-Mont-d'Or (FR)
(74) Mandataire: Cabinet Plasseraud
(86) Numéro de dépôt international: PCT/EP2012/070350
(87) Numéro de publication internationale: WO 2013/053929

(56) Documents cités:
- WO-A1-03/059215
- DE-A1- 19 751 733
- FR-A1- 2 887 436
- FR-A1- 2 903 297
- FR-A1- 2 944 431
- US-A1- 2010 324 359

## Description

### Domaine technique

Le domaine technique de l'invention est celui des implants chirurgicaux destinés à être mis en place autour d'un segment du tube digestif pour le rétrécir, par exemple entre la partie abdominale de l'oesophage et l'estomac. L'étranglement ainsi obtenu crée en amont de l'estomac, une poche de rétention du bol alimentaire de beaucoup plus faible volume que l'estomac, de sorte que les signaux induisant la satiété sont émis précocement chez le patient. De tels implants sont donc utilisés pour le traitement de l'obésité.

### Art antérieur et problèmes techniques

Classiquement, ces implants peuvent être du type de ceux comprenant un corps creux allongé et gonflable au moyen d'un fluide approprié, par exemple l'eau physiologique. Ce corps creux est mis en place, en position ouverte, par le chirurgien autour d'un segment du tube digestif situé e.g. entre la partie abdominale de l'oesophage et l'estomac. Le chirurgien relie ensuite l'une à l'autre les deux extrémités de l'implant pour verrouiller l'anneau ainsi formé à une dimension correspondant à un niveau basal d'étranglement de l'estomac. Le chirurgien peut ensuite régler autant que de besoin, le niveau d'étranglement, et donc le sevrage du patient, par gonflage/dégonflage de l'anneau fermé implanté, depuis l'extérieur du corps du patient au moyen d'une seringue et d'une chambre à septum implantable reliée à l'anneau par un cathéter, ce réglage étant fait lors d'une simple hospitalisation.

Les problèmes techniques qui se posent vis-à-vis de ces implants gastriques gonflables et ajustables sont notamment ceux évoqués ci-après:
a. La facilité d'implantation pour le chirurgien qui influe directement sur le confort et la santé du patient.
b. En prolongement du problème (a), la possibilité de réaliser l'implantation par coelioscopie est un atout appréciable.
c. Biocompatibilité pérenne de l'implant.
d. Les sollicitations dues à l'ingestion d'aliments sont susceptibles de provoquer le déplacement de l'anneau. Cela peut être évité au moyen de sutures, mais idéalement l'anneau implanté devrait avoir un positionnement stable, sans avoir recours à ce moyen agressif.
e. Le fait d'être visible aux rayons X (radio-opacité) est un atout recherché pour l'implant.
f. Il est également souhaitable que ce type d'implant gastrique gonflable ne soit pas sujet à un phénomène d'obturation, par la paroi interne, de l'ouverture de gonflage connectée au cathéter et disposée sur la paroi externe de la bande creuse, notamment lors du dégonflage de cette bande creuse par aspiration du fluide de gonflage.
g. La sécurité et la fiabilité de la fermeture en anneau de la bande creuse autour de l'estomac du patient, est aussi un élément déterminant de l'efficacité de l'implant. Il est donc souhaitable d'assurer à cette fin, un verrouillage optimal de cette fermeture.
h. Les impératifs de production en série selon des normes élevées de qualité et au meilleur coût, sont également au coeur de la problématique de la présente invention.
i. Il importe pour assurer par le gonflage une déformation centripète de l'anneau gastrique fermé, que le dos ou la paroi externe soit renforcé par rapport à la paroi interne dont la face externe est en contact avec le tube digestif après implantation.

Le brevet français FR2887436-B1 divulgue une ceinture gastrique gonflable comprenant un corps tubulaire allongé en matière souple, élastiquement déformable, ce corps définissant une chambre étanche gonflable et présentant un dos et une face de travail. Cette ceinture comporte également des moyens de liaison disposés en relation avec deux extrémités du corps tubulaire et permettant de fermer la ceinture gastrique sous la forme d'un anneau, la face de travail étant disposée à l'intérieur de l'anneau. Cette ceinture comprend enfin un cathéter de gonflage destiné à être raccordé à des moyens de gonflage et raccordé de façon étanche, à la chambre gonflable via un orifice de gonflage aménagé dans le dos du corps tubulaire allongé. La face interne du dos du corps tubulaire présente huit protubérances destinées à offrir un appui à la paroi interne de la chambre au voisinage de l'orifice de gonflage aménagé dans le dos du corps tubulaire.
La ceinture gastrique selon le brevet français FR2887436-B1 est perfectible au regard de tout ou partie des problèmes techniques (a) à (i) mentionnés ci-dessus, en particulier en ce qui concerne (a) la facilité de mise en place de l'implant autour de l'estomac par le chirurgien et plus particulièrement, pour l'introduction du cathéter dans les passants permettant de former l'anneau autour de l'estomac par bouclage.

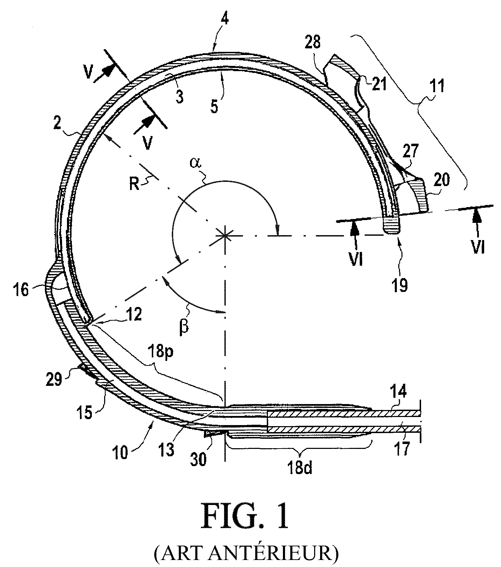

Le brevet français FR2903297 divulgue une bande-anneau gastrique gonflable selon le préambule de la revendication 1 annexée, plus précisément une ceinture gastrique 1 comprenant un corps tubulaire 2 définissant une chambre 3 étanche et gonflable présentant en élévation vu du dessus, une forme générale en C, comme montré sur la figure 1 annexée qui représente une coupe selon le plan longitudinal médian de la ceinture gastrique selon le brevet français FR2903297, au repos et à l'état dégonflé, le corps tubulaire 2 a une forme générale plate et une section droite transversale rectangulaire. La ceinture gastrique comporte également un cathéter de gonflage 14 reliant un orifice de gonflage 16 ménagé dans le dos 4 du corps tubulaire 2, à des moyens de gonflage. L'une des extrémités du cathéter 14 est destinée à être passée au travers de deux arceaux 20,21 disposés sur le dos 4 du corps tubulaire 2 - sur sa partie d'extrémité libre courbe- pour former un anneau par bouclage autour de l'estomac. La partie d'extrémité 18p du cathéter 16 comprend deux moyens de blocage anti-retour 22 en forme de sapin destinés à coopérer avec les arceaux 20,21 pour verrouiller l'anneau en position fermée. La partie d'extrémité 18p du cathéter 16 qui comprend ces sapins anti-retour 22 est cintrée sur un secteur angulaire β de 45 à 75° de même rayon de courbure et dans le prolongement de l'arc de cercle formé par le corps tubulaire sur un secteur tubulaire α compris entre 160 et 230 ° C.
Cette ceinture gastrique selon le brevet français FR2903297-B1 est également perfectible au regard de tous les problèmes techniques a à i susmentionnés, notamment au regard de la facilité de pose autour de l'estomac par le chirurgien, notamment pour l'enfilage de l'extrémité libre proximale du cathéter dans les passants prévus sur la partie d'extrémité distale du corps creux destiné à former un anneau par bouclage.
La demande WO03/059215A1 décrit un anneau de gastroplastie préformé en "C" au repos, réalisé en matériau élastomère et relié à un cathéter de gonflage. Cet anneau torique relativement rigide, est destiné à être fermé autour de l'estomac du patient. L'anneau comporte une chambre de compression annulaire gonflable et délimitée par un renfort dorsal (externe) en "U" sur lequel sont surmoulées des parois dites "latérales" (partie interne) pour former un ensemble présenté comme "monobloc", mais qui ne l'est pas réellement, car composé de plusieurs pièces surmoulées (discontinuité des matériaux). Le renfort dorsal est dans un matériau à dureté supérieure à celle de la partie interne de l'anneau. Les moyens de fermeture à un seul verrou, consistent en un moyen femelle formé par une bague solidaire de l'extrémité de l'anneau et par un moyen mâle formé par un embout solidaire de l'autre extrémité de l'anneau. Cette bague et cet embout ne sont pas, au repos, dans le prolongement de l'anneau préformé en "C" .
Cet anneau présente l'inconvénient d'être non réellement monobloc, difficile à fabriquer, et de ne posséder qu'un seul verrou de fermeture, ce qui augmente les risques d'ouverture intempestive et les complications associées.
Cet anneau a, de plus, une épaisseur non négligeable et une certaine rigidité due à son procédé de fabrication par préformage. Sa pose autour de l'estomac est une opération délicate et risquée car elle nécessite le passage de l'intégralité de l'anneau sous (derrière) l'estomac avec les risques d'endommagement de la rate et/ou de la veine porte à proximité de l'estomac. En outre, l'embout rigide de fermeture de cet anneau est saillant en position verrouillée et comporte de plus des languettes rigides, de sorte que les risques de blessure des organes périphériques de l'estomac du patient sont réels.

Dans ce contexte, la présente invention a pour objectif d'améliorer les implants chirurgicaux du type de ceux décrits dans les brevets FR2887436-B1, FR2903297-B1 et WO 03/059215 A1, s'agissant des problèmes techniques mentionnés ci-dessus.

En particulier, la bande-anneau gastrique gonflable et ajustable selon l'invention vise assurer le plus parfaitement possible le confort et la sécurité du patient, notamment en facilitant le travail du chirurgien pour la pose rapide et fiable de l'anneau autour d'un segment donné du tube digestif. L'anneau fermé, ainsi mis en place, se doit d'être stable quant à son positionnement, non agressif et efficace.

Pour optimiser le gonflage, donc l'ajustement du niveau d'étranglement, la bande anneau selon l'invention se doit de posséder un renfort de la paroi externe dorsale pour que le gonflage s'opère au mieux de manière centripète.

Il est également souhaitable que le dégonflage ne provoque pas d'occlusion de l'ouverture de l'orifice de gonflage de la paroi externe par la paroi interne, du fait d'un phénomène de dépression lors du dégonflage.

L'optimisation de la fabrication industrielle avec une haute exigence de qualité et d'économie est également visée par la présente invention.

### Brève description de l'invention

Ces objectifs parmi d'autres sont atteints par la présente invention qui concerne une bande-anneau gastrique gonflable, ajustable destinée à être mise en place autour d'un segment du tube digestif pour le rétrécir, ladite bande-anneau comprenant les caractéristiques définies dans la revendication 1 annexée.

### Définitions

*L*'*extrémité proximale* du tube souple est son extrémité libre la plus proche de l'extérieur du corps du patient dans lequel l'implant est mis en place, c'est-à-dire l'extrémité libre destinée à être reliée au moyen de gonflage/dégonflage.

L'*extrémité distale* du tube souple est l'extrémité opposée à son extrémité proximale, cette extrémité distale est reliée à l'extrémité proximale de la bande creuse de la bande-anneau selon l'invention.

L'*extrémité proximale* de cette bande creuse est donc l'extrémité de la bande creuse la plus proche du tube souple.

L'*extrémité distale* de la bande creuse est son extrémité libre opposée à son extrémité proximale.

Par *dureté* D1, D2, on désigne la dureté Shore A du ou des matériaux constitutifs de la bande-anneau et en particulier de la bande souple creuse.

Le terme *bande souple* désigne un dispositif sensiblement mince lorsqu'il est en position de repos, c'est -à-dire non gonflée.

Par *monobloc,* on entend un produit fait d'une seule pièce, de préférence obtenu à l'aide d'un moule à multi-injection séquentielle garantissant la fusion des matériaux moulés constituant le produit, de préférence des silicones, et la continuité desdits matériaux. Un tel produit monobloc est obtenu sans surmoulage, c'est-à-dire avec un continuum de matière moulée.

### Caractéristiques préférées et avantages de l'invention

La forme particulière courbe puis rectiligne de la bande-anneau selon l'invention et en particulier de la bande creuse, au repos et sans engagement du tube et de la bande souple creuse l'un dans l'autre pour former l'anneau, simplifie sa mise en place autour de l'estomac, de même que son bouclage par engagement de l'extrémité libre proximale du tube souple dans le ou les passants (ou réciproquement) est favorisée par la forme rectiligne de la partie distale de la bande creuse. La partie rectiligne de la partie distale de la bande souple creuse est suffisamment longue pour porter au moins un, de préférence au moins deux passants sur la paroi externe et pour aider le chirurgien lors la fermeture de la bande-anneau, par passage de l'extrémité libre du tube dans au moins un, de préférence au moins deux passants. La pose de la bande-anneau se fait en plusieurs étapes sous coelioscopie et sous anesthésie générale :
1) l'extrémité proximale du tube souple est passée dans la région latérale droite de l'estomac, puis sous l'estomac (postérieurement à l'estomac) pour ressortir dans la région latérale gauche de l'estomac (patient positionné face au chirurgien pratiquant l'opération),
2) le tube souple est déroulé par une traction sur la partie proximale du tube vers l'extérieur du corps du patient, de sorte à amener la bande souple creuse autour de l'estomac,
3) la partie rectiligne de la bande souple creuse est positionnée, rabattue sur la face antérieure de l'estomac, de sorte que le ou les passants sur sa paroi externe soi(en)t face au chirurgien pratiquant l'opération,
4) l'extrémité proximale du tube souple est enfilée en un seul geste rectiligne au travers des deux passants en vue de fermer la bande-anneau,
5) l'extrémité proximale du tube est tirée jusqu'à ce que le ou les crans de la partie distale du tube viennent s'engager dans le ou les passant(s) de la paroi externe de la partie rectiligne de la bande souple creuse.
Sous coelioscopie, le préformage de la bande souple creuse présente l'avantage de permettre un positionnement naturel de la bande-anneau autour de l'estomac, sans que le médecin ait besoin de rabattre la partie distale de la bande souple creuse sur la face antérieure de l'estomac. Ceci évite donc un geste au chirurgien, facilitant ainsi la pose et assurant également la sécurité du patient car la zone opérée est particulièrement délicate et la minimisation des mouvements des outils opératoires minimise, de fait, les risques opératoires.

Par ailleurs, toujours sous coelioscopie, le fait que la bande souple creuse ait une partie rectiligne dans sa région distale présente l'avantage de faciliter la pose de la bande-anneau et particulièrement sa fermeture. L'enfilage de l'extrémité proximale du tube souple au travers du ou des passants de la partie rectiligne de la paroi externe de la bande creuse peut se faire en un seul geste, en une seule fois lorsque ces derniers sont dans la même axe. En effet, si la partie distale de la bande souple creuse était courbe, pour fermer la bande-anneau, le chirurgien pratiquant l'opération (sous coelioscopie) devrait passer l'extrémité proximale du tube dans le premier passant selon un axe puis récupérer l'extrémité proximale du tube pour la faire passer dans le second passant selon un autre axe. Comme évoqué précédemment, plus on réduit les gestes opératoires, plus la pose est facile et plus la sécurité du patient est assurée. Ainsi, il apparait clairement que le fait d'avoir deux passants sur un même axe, celui de la partie rectiligne de la partie distale de la bande souple creuse, facilite la pose et minimise les risques opératoires.

Enfin, le fait que la partie rectiligne dans la partie distale de la bande souple creuse, sur sa paroi externe, porte, dans un mode de réalisation préféré, au moins deux passants, permet également une sécurité supplémentaire pour le patient. D'une part, la bande-anneau fermée avec un seul passant pourrait s'ouvrir, ce qui présente un risque pour le patient et nécessite une opération chirurgicale supplémentaire. D'autre part, la bande-anneau fermée avec un seul passant se vrille et risque de se retrouver à l'envers autour de l'estomac, la paroi externe se retrouvant alors en contact direct avec l'estomac. La bande-anneau souple pourrait alors endommager l'estomac avec les passants ou les crans ou entraîner une érosion ou une migration dans l'estomac, ce qui est dangereux.

Avantageusement, la longueur de la partie rectiligne de la partie distale de la bande souple creuse, au repos, c'est-à-dire non gonflée, est comprise entre 10 et 40 %, plus préférentiellement entre 17 et 35%, plus préférentiellement encore entre 21 et 33% de la longueur totale de la bande souple creuse.

Par exemple, la longueur de la partie rectiligne est de 4,5 cm et la longueur de la bande souple est de 18,5 cm. La longueur de la partie rectiligne représente donc 24,3% de la longueur de la bande souple creuse.

La longueur de la bande est mesurée de l'extrémité distale à l'extrémité proximale de la bande souple creuse.

Avantageusement, la distance entre les deux passants (prise entre la partie proximale du passant le plus proche de l'extrémité proximale de la bande et la partie distale du passant le plus proche de l'extrémité distale de la bande) portés par la partie rectiligne de la partie distale de la bande souple creuse, au repos, c'est-à-dire non gonflée, est comprise entre 10 et 40 %, plus préférentiellement entre 17 et 35%, plus préférentiellement encore entre 21 et 33% de la longueur totale de la bande souple creuse. Par exemple, la distance entre les deux passants est comprise entre 16 et 34mm ± 1 mm et la longueur de la bande souple est de comprise entre 108 et 122 mm ± 1 mm. La distance entre les deux passants représente donc entre 12 et 32 % de la longueur de la bande souple creuse

Selon une modalité préférée de l'invention, l'axe de cette partie sensiblement rectiligne s'étendant jusqu'à l'extrémité libre distale de la bande souple creuse est sécant à la zone d'extrémité distale du tube.

Pour améliorer encore l'efficacité de cette forme courbe à prolongement rectiligne de la bande souple creuse, il est préférable que la zone d'extrémité distale du tube présente, au repos et sans engagement du tube et de la bande creuse l'un dans l'autre pour former l'anneau, une partie sensiblement rectiligne.
Dans cette dernière variante avantageuse de réalisation, il est préférable qu'au repos et sans engagement du tube et de la bande creuse l'un dans l'autre, l'angle A que forme la partie sensiblement rectiligne distale de la bande creuse et la partie sensiblement rectiligne distante du tube est défini comme suit en °, selon un ordre croissant de préférence:
▪ A ≤ 60
▪ A ≤ 50
▪ 20 ≤ A ≤ 50
▪ 40 ≤ A ≤ 90
▪ A = 45.
Pour la mesure de cet angle A, on procède par exemple de la façon suivante : la bande souple creuse est amenée sans contrainte dans une position où la bande souple creuse est sensiblement perpendiculaire, à quelques centimètres, par exemple entre 1 et 5 cm, d'un support plan (e.g. le plateau d'une table). Cette bande souple creuse est ensuite lâchée sur le support en faisant en sorte que le tube repose sur le support selon une direction sensiblement rectiligne dans le prolongement de l'extrémité proximale de la bande souple creuse, pour ne pas contraindre cette dernière à être dans une position autre que celle qu'elle prend après avoir été lâchée, comme expliqué ci-avant.

Dans un mode particulièrement préféré de réalisation, la zone d'extrémité proximale de la bande creuse est sensiblement rectiligne.

Suivant une autre caractéristique remarquable de l'invention, qui facilite la fermeture de la bande-anneau, la partie préformée courbe est au moins partiellement circulaire et le secteur angulaire S défini à partir du centre de ce cercle, entre l'extrémité proximale et l'extrémité distale de la bande, est défini comme suit en ° dans un ordre croissant de préférence:
S > 230; S ≥240; S ≥250; S ≥260; S ≥270; S ≥280; S ≥290; 330 ≥S ≥ 300; 320 ≥S ≥ 310.

De manière remarquable le rayon de courbure R de ce cercle est compris dans les intervalles suivants donnés en mm et dans un ordre croissant de préférence [10-21] ; [13-20]; [14-19]; [15-18]; [17-18].

Dans une forme préférée de réalisation de l'invention, la bande souple creuse de la bande-anneau présente un rapport longueur/largeur compris entre 4,5 et 5,9.

Par exemple, la largeur de la bande souple creuse de la bande-anneau est d'environ 22 mm ± 1mm et la longueur de la bande souple creuse de la bande-anneau est comprise entre 108 mm et 122 mm ± 1mm, soit un rapport longueur/largeur compris entre 4, 65 et 5,85.

Pour améliorer la qualité de l'ajustement de l'étranglement de l'estomac par gonflage de la bande-anneau gastrique, il est prévu conformément, à une caractéristique notable de l'invention que la bande souple creuse soit monobloc et soit réalisée à partir d'au moins deux matières M1, M2, la matière M1 constituant au moins en partie la paroi externe et la matière M2 constituant au moins en partie la paroi externe. De préférence, M1, M2 sont choisies parmi les élastomères silicone, se différenciant au moins par leurs duretés respectives D1, D2 avec D1 > D2, et plus particulièrement dans un ordre croissant de préférence, D1 > 1,1(D2) ; D1 > 1,2(D2) ; 2(D2) > D1 > 1,3(D2).

Dans une forme de réalisation préférée, la bande souple creuse telle que décrite ci-dessus constituée de deux matières M1 et M2 et comportant sur sa paroi externe dans sa partie rectiligne distale au moins deux passants est monobloc.

Une des caractéristiques essentielles de l'invention repose sur le choix des dimensions et des matériaux constituant la bande-anneau qui lui offrent une grande souplesse. Cette souplesse est telle que la bande-anneau peut être introduite avec une pince de préhension Karl Storz ® pour la coelioscopie au travers d'un coelio trocart 12mm de marque Covidien®. Ceci n'est pas possible avec les bandes-anneaux de l'art antérieur qui sont trop rigides et ne peuvent passer que dans des trocarts supérieurs au trocart de 12mm. Cette souplesse est avantageuse à plusieurs niveaux :
- elle permet l'utilisation de trocart de 12mm seulement ce qui réduit les risques d'éventration, de hernie ou de mauvaise cicatrisation lors de l'opération par laparoscopie,
- elle permet de réduire également les risques d'endommagement des tissus et organes environnant l'estomac lors de sa mise en place autour de celui-ci et une fois placée autour de celui-ci.
Le terme *souple* utilisé dans la présente demande correspond à la souplesse telle que définie ci-dessus. Ainsi, la bande creuse selon l'invention peut être caractérisée de bande souple puisqu'elle peut être introduite via lesdites pinces de laparoscopie dans ledit trocart de 12mm.

Il est important que la bande anneau gastrique une fois implantée soit visible par le chirurgien au moyen de dispositif usuel d'imagerie médicale telle que les rayons X. Pour ce faire, il est donc avantageux que la bande-anneau selon l'invention soit en partie radio-opaque. De préférence, les arêtes longitudinales de la paroi externe sont protubérantes, et, plus préférentiellement encore, sont au moins en partie radio-opaque, notamment aux rayons X.

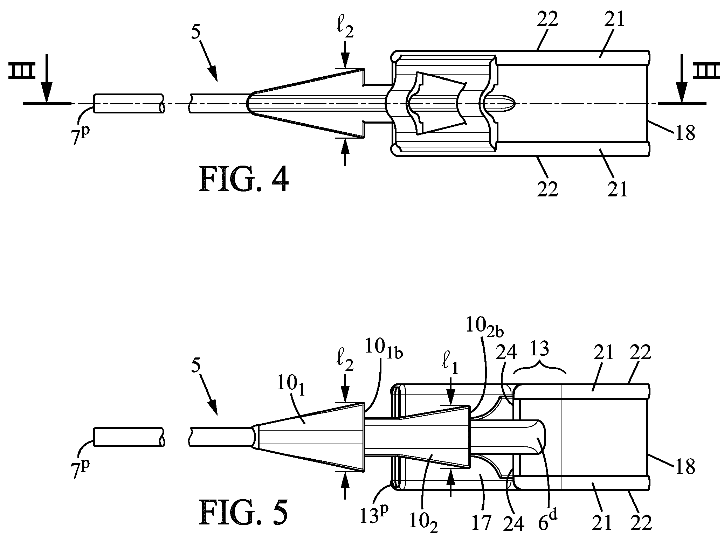

Une fois l'extrémité libre proximale du tube souple engagée dans la partie d'extrémité libre distale sensiblement rectiligne de la bande souple creuse et refermée pour former un anneau selon le principe du noeud coulant, ledit anneau doit pouvoir être verrouillé en position fermée. A cette fin, la zone d'extrémité distale du tube comprend au moins deux crans disposés en série 10₁ & 10₂ et en forme de queues d'aronde dont les bases 10_{1b} & 10_{2b} sont de largeurs différentes, la largeur l₁ du cran 10₂ le plus proche de l'extrémité distale étant inférieure ou égale à la largeur l₂ de l'autre cran 10₁ (Figure 5)
Comme énoncé ci-dessus, la partie rectiligne distale de la bande souple creuse permet de guider le chirurgien lors de la fermeture de l'anneau gastrique et faciliter ainsi cette fermeture. Le ou les passants étant sur la paroi externe de la bande souple creuse, la fermeture de l'anneau se fait lorsque les crans du tube sont passés au-delà de leur(s) passant(s) correspondant(s) respectif(s). Ainsi, le tube vient recouvrir la partie (rectiligne au repos et curviligne lorsque la bande anneau est en place autour de l'estomac) distale de la bande souple creuse. Ceci a pour avantage supplémentaire de limiter les risques de perforation ou d'endommagement des organes périphériques à l'estomac lors de la pose et de la fermeture de l'anneau. Le tube de gonflage des dispositifs de l'art antérieur se placent le plus souvent sensiblement perpendiculaire à la partie distale de l'anneau ou du moins non tangentiel à cette dernière. Pour la fermeture de la bande-anneau selon l'invention, le tube va ainsi longer la paroi externe de la partie rectiligne de la bande souple creuse jusqu'à un verrouillage en position fermée, par exemple les crans en queues d'aronde étant passés au travers du ou des passants.
Dans une forme préférée de réalisation de l'invention, lorsque la bande-anneau est fermée, en position gonflée ou non, les extrémités respectives proximale et distale de la bande souple creuse viennent en contact l'une avec l'autre. Ainsi, lorsque la bande-anneau est placée autour de l'estomac, ce dernier n'est en contact qu'avec la paroi interne de la bande souple creuse, sans aucune protubérance. Le dispositif une fois forme est ainsi atraumatique pour l'estomac et les organes environnants.

Selon une autre modalité intéressante de l'invention, l'ouverture de gonflage de la bande souple creuse est ménagée dans sa paroi externe au niveau de la zone d'extrémité proximale de celle-ci.

En outre, cette paroi externe présente avantageusement, sur sa face interne, des évidements, de préférence des rainures, plus préférentiellement encore des rainures longitudinales parallèles et/ou des rainures transversales parallèles, au moins une partie de ces évidements étant disposée de part et d'autre de l'ouverture de gonflage, lesdits évidements étant destinés à empêcher une occlusion de l'ouverture de gonflage par la paroi interne de la bande souple creuse en regard de cette ouverture, notamment lors du dégonflage.

De préférence, au moins une des arêtes longitudinales de la bande souple creuse est émoussée ou arrondie.

De préférence, la largeur de la paroi interne de la bande souple creuse selon l'invention, en position gonflée ou non, n'excède pas la largeur de la paroi externe de la bande souple creuse, c'est-à-dire qu'elle ne dépasse pas de chaque côté de cette dernière.

De préférence, la bande-anneau est obtenue par moulage à l'aide d'un moule dont les caractéristiques définissent ladite bande-anneau.

De préférence, la bande-anneau selon l'invention est obtenue par multi-injection séquentielle au sein d'un même moule.

L'invention concerne également le moule en lui-même et le procédé d'obtention de la bande-anneau par moulage à l'aide dudit moule, le moulage étant de préférence monobloc par multi-injection séquentielle au sein d'un même moule.

Dans le moule, l'empreinte de la bande-anneau est caractérisée par un angle A (en degré) entre la partie sensiblement rectiligne distale de la bande souple creuse et la partie sensiblement rectiligne distale du tube, est tel que, selon un ordre croissant de préférence:
▪ A ≤ 60
▪ A ≤ 50
▪ 20 ≤ A ≤ 50
▪ 40 ≤ A ≤ 90
▪ A = 45.

Dans le moule, l'empreinte de la partie préformée courbe de la bande-anneau est au moins partiellement circulaire et le secteur angulaire S défini à partir du centre de ce cercle entre l'extrémité proximale et l'extrémité distale de la bande souple creuse, est défini comme suit dans un ordre croissant de préférence :
▪ S > 230,
▪ S ≥240,
▪ S ≥250,
▪ S ≥260,
▪ S ≥270,
▪ S ≥280,
▪ S ≥290,
▪ 330 ≥S ≥ 300,
▪ 320 ≥S ≥310.

Selon un autre de ces aspects, l'invention concerne l'utilisation, dans une bande-anneau du type de celle selon l'invention, d'évidements de préférence de rainures, plus préférentiellement encore de rainures longitudinales parallèles dont au moins une partie est disposée de part et d'autre de l'ouverture de gonflage; ces évidements étant ménagés dans la face interne de la paroi externe comprenant également l'ouverture de gonflage, pour empêcher une occlusion de l'ouverture de gonflage par la paroi interne de la bande souple creuse en regard de cette ouverture, notamment lors du dégonflage.

L'invention vise également un nécessaire pour la réalisation par voie chirurgicale d'un rétrécissement d'un segment du tube digestif, de préférence de l'estomac, comprenant:
- une bande-anneau telle que définie dans la présente demande;
- et un module de gonflage équipé d'un septum et destiné à être relié à l'extrémité proximale du tube souple, pour permettre l'injection ou l'extraction in situ d'un fluide de gonflage pour le gonflage ou le dégonflage de la bande souple creuse.

Prise dans tous ces aspects, la présente invention constitue une avancée significative dans le domaine des implants gastriques pour le traitement de l'obésité. Les acquis les plus marquants se situent au niveau de l'assistance à la chirurgie qui renforce la sécurité et le confort de fabrication industrielle par la réalisation économique et performante d'un ensemble moulé monobloc comprenant au moins deux, voire au moins trois matières, *e.g.* à l'aide de moules multi-injection.

### Description détaillée de l'invention

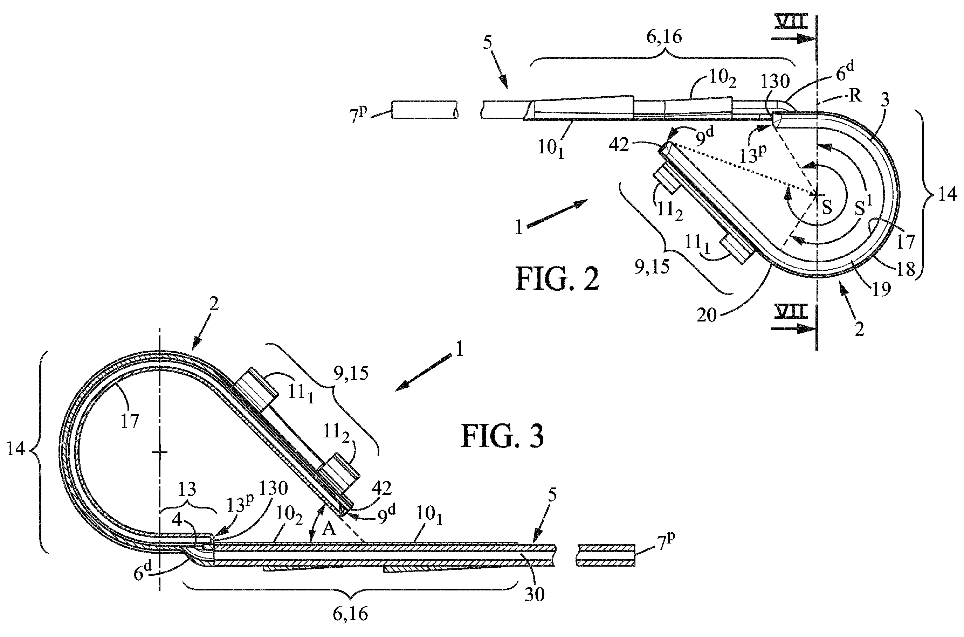

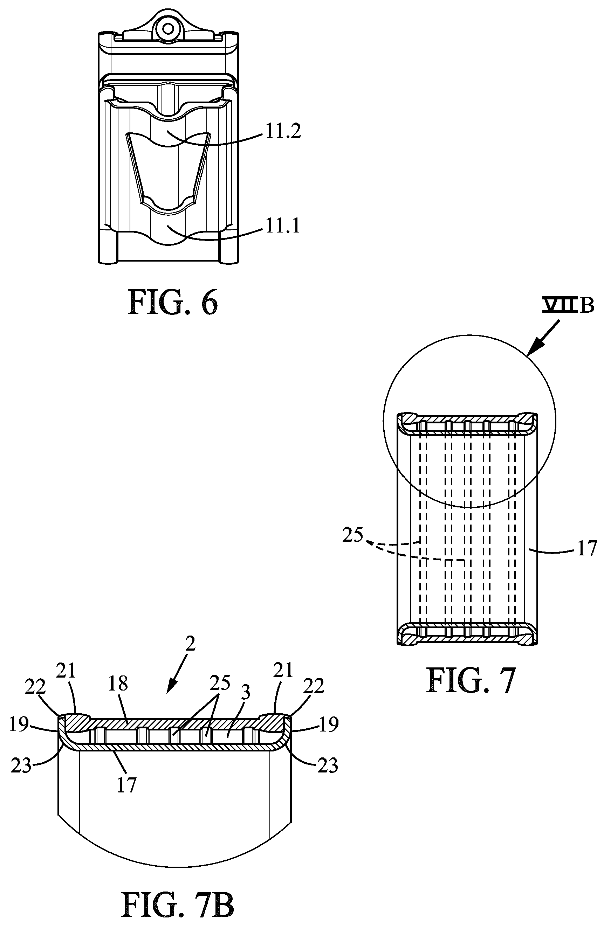

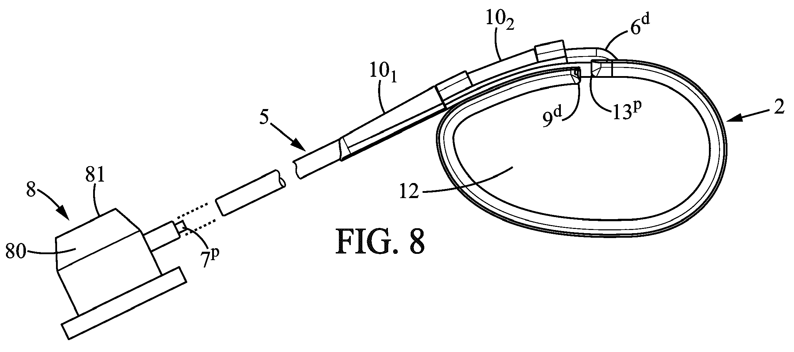

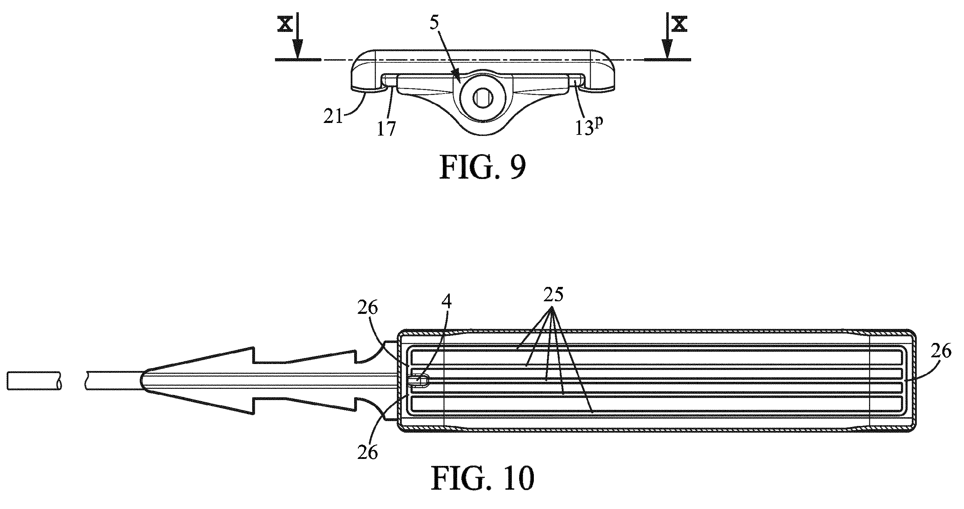

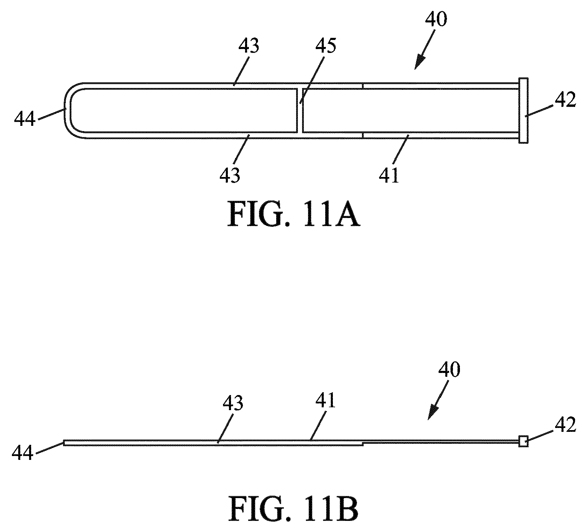

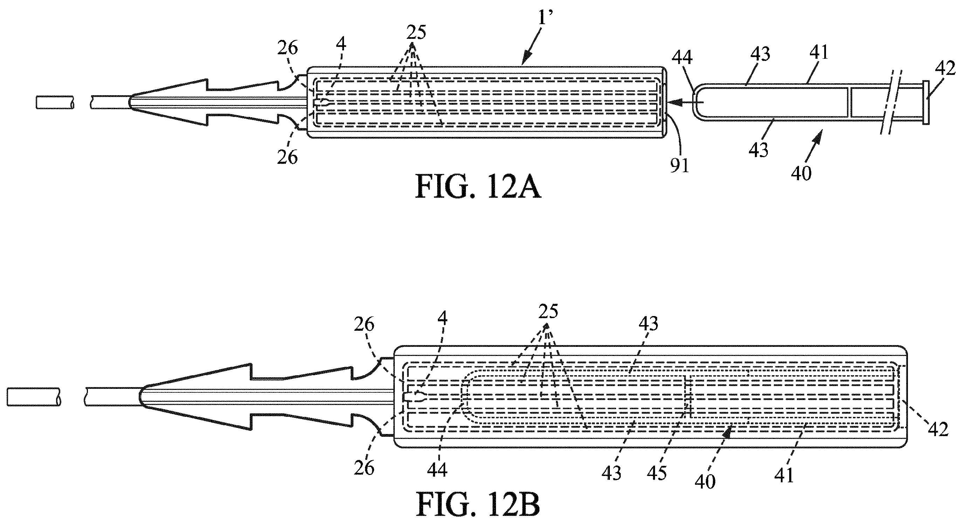

La description qui suit d'un mode préféré de réalisation de la bande-anneau selon l'invention, fera ressortir d'autres de ses caractéristiques remarquables. Cette description détaillée est faite en référence aux figures annexées dans lesquelles :
- **la** **figure 2** est une vue de côté de la bande-anneau gastrique selon l'invention, au repos, à l'état dégonflé et en position ouverte (sans engagement de la bande souple creuse et du tube l'un dans l'autre pour former une boucle,
- **la** **figure 3** est une vue en coupe de la figure 2 selon le plan longitudinal médian (ligne III-III de la figure 4),
- **la** **figure 4** est une vue de dessous de la figure 2,
- **la** **figure 5** est une vue de dessus de la figure 2,
- **la** **figure 6** est une vue du côté gauche de la figure 2,
- **la** **figure 7** est une vue en coupe transversale selon la ligne VII-VII de la figure 2,
- **la** **figure 7B** est une loupe VII de la figure 7,
- **la** **figure 8** est une vue de face de la bande-anneau selon l'invention en position fermée et à l'état dégonflé, l'extrémité proximale du tube souple de cette bande-anneau étant reliée à un module de gonflage/dégonflage équipé d'un septum,
- **la** **figure 9** est une vue de côté de la figure 2 dans laquelle la bande-anneau est maintenue étendue à plat et dans laquelle la paroi interne a été supprimée pour faire apparaitre la face interne de la paroi externe de la bande-anneau et en particulier les rainures anti-collapse que comporte ladite face interne,
- **la** **figure 10** est une vue en coupe longitudinale selon la ligne X/X de la figure 9.
- **la** **figure 11A** est une vue de face d'un insert radio-opaque destiné dans la bande-anneau gastrique selon l'invention.
- **la** **figure 11B** est une vue en côté de la figure 11A.
- **la** **figure 12A** est une vue de dessous (paroi interne) de la bande-anneau gastrique selon l'invention ouverte à son extrémité distale et avant finition, ainsi que de l'insert radio-opaque des figures 11A&11B avant son introduction dans la bande-anneau.
- **la** **figure 12B** est une vue de la figure 12A après introduction de l'insert radio-opaque des figures 11A&11B, dans la bande-anneau.

La bande-anneau gastrique selon l'invention, est désignée par la référence générale 1 sur les figures annexées.

Cette bande-anneau 1 gonflable et ajustable autour d'un segment du tube digestif pour le rétrécir par étranglement comporte une bande souple creuse 2 réalisée en un matériau élastique, par exemple un élastomère silicone. Cette bande creuse 2 est allongée et présente une forme sensiblement rectangulaire en section transversale droite. Cette bande souple creuse 2 comporte une partie 13 d'extrémité proximale 13^{p} sensiblement rectiligne puis une partie courbe 14 se prolongeant par une partie 15 d'extrémité distale 9^{d} sensiblement rectiligne et porteuse de moyens de fermeture 11.

La partie d'extrémité proximale 13^{p} de la bande souple creuse 2 est reliée à un tube souple de gonflage 5 et plus précisément à la partie 16 d'extrémité distale 6^{d} rectiligne dudit tube souple 5 équipé de moyens de fermeture 10, complémentaires aux moyens de fermeture 11 de la bande souple creuse 2 portés, de préférence sur la paroi externe de la partie rectiligne distale de la bande creuse.

Le tube souple 5 de gonflage présente une extrémité proximale 7^{p} libre destinée à être connectée à des moyens de gonflage 8 comprenant eux-mêmes un module de gonflage 80 montré sur la figure 8 et équipé d'un septum et permettant l'injection de liquide de gonflage ou l'aspiration de liquide de gonflage à l'aide d'une seringue.

La bande souple creuse 2 :
La bande souple creuse 2 comprend une paroi interne 17 et une paroi externe 18 reliée l'une à l'autre par deux parois latérales 19 et deux parois terminales, respectivement proximale 130 et distale 90, qui définissent une chambre 30 sensiblement parallélépipédique.

Cette bande creuse 2 est en forme générale de crochet avec une partie préformée courbe 14, par exemple sensiblement circulaire, caractérisée par un secteur angulaire S défini à partir du centre de la partie circulaire entre l'extrémité proximale 13^{p} et l'extrémité distale 9^{d} de la bande 2 compris entre 320 et 310°.

S'agissant de la partie préformée courbe circulaire proprement dite, son secteur angulaire S¹ qui s'étend entre l'intersection de l'axe VII-VII avec la bande 2 creuse au bout de la zone 13 d'extrémité proximale 13^{p} et le début 20 de la partie 15 d'extrémité libre distale 9^{d} de cette bande 2 est compris entre 180 et 270°.

Par ailleurs, l'axe de la partie 15 d'extrémité libre distale 9^{d} rectiligne de la bande souple creuse 2 est sécant à la partie 16 d'extrémité distale 6d rectiligne du tube souple 5, de manière à faciliter l'enroulement de la bande creuse 2 autour de l'estomac par le chirurgien.

Comme montré notamment sur la figure 10, la face interne de la paroi externe 18 présente des rainures longitudinales 25 parallèles et deux rainures transversales 26 parallèles et localisées aux extrémités de cette face interne. Par ailleurs, une ouverture 4 de gonflage reliant le tube souple 5 à l'intérieur de la bande souple creuse 2, est ménagée dans la paroi externe 18 au voisinage de l'extrémité proximale 13^{p}. La rainure transversale 26 prévue à cette extrémité est disposée de part et d'autre de l'ouverture 4, de même que les parties terminales proches des rainures longitudinales 25. Ces rainures 25,26 permettent notamment d'empêcher une occlusion de cette ouverture 4 lors du dégonflage du fait d'un phénomène de dépression venant appliquer la paroi interne 17, en regard de l'ouverture 4 pour la fermer.

Les moyens 11 de fermeture disposés sur la paroi externe 18 de la bande souple creuse 2 sont dans cet exemple préféré de réalisation des passants 11₁,11₂. Ces passants sont de préférence au moins au nombre de deux. Il peut bien évidemment s'agir d'autres moyens adaptés pour assurer une bonne fermeture de l'anneau gastrique.
De plus, toujours selon l'exemple illustré, afin de favoriser ce passage du tube souple et réduire, autant que faire se peut, les efforts nécessaires à ce geste chirurgical, les passants présentent, sur leur face interne, une série de stries qui viennent réduire la surface de contact du tube souple avec le passant correspondant, de manière à réduire les forces de friction. Ces stries s'étendent parallèlement à l'axe longitudinal de la bande souple creuse 2 et dans la direction d'introduction du tube souple 5.
La bande souple creuse et les passants qu'elle porte sont monobloc, c'est-à-dire qu'ils ne forment qu'un.
Comme il est possible de le voir sur la figure 3, la distance entre les passants (prise entre l'extrémité proximale du passant proximal 11₁ le plus proche de l'extrémité proximale de la bande 13^{p} et l'extrémité distale du passant distal 11₂ le plus proche de l'extrémité distale 9^{d} de la bande) portée par la partie rectiligne de la partie distale de la bande souple creuse représente environ 38 mm. Ceci représente environ 21% de la longueur totale de la paroi interne de la bande creuse prise entre l'extrémité proximale 13^{p} et l'extrémité distale 9^{d}.

La bande souple creuse 2 comporte également deux bandes longitudinales radio-opaques 21 parallèles, au voisinage des arêtes longitudinales 22 de la paroi externe 18. Comme montré sur la figure 5, une bande radio-opaque 24 est prévue également sur le bord transversal proximal de la bande souple 2 creuse.

Il est à noter que ces arêtes longitudinales 22 de même que les arêtes correspondantes 23 de la paroi interne 17 de la bande souple creuse 2 sont émoussées, voire arrondies de manière à ne pas présenter d'angles saillants susceptibles de blesser le patient.

La bande souple creuse 2 a pour caractéristique d'avoir une paroi externe 18 réalisée en un matériau élastomère, -par exemple silicone biocompatible - M1 de dureté D1, tandis que la paroi interne 17 est réalisée en un matériau élastomère -par exemple silicone biocompatible- M2 de dureté D2 inférieure à D1. En pratique D1 est par exemple égal à 0,5 x D2. La paroi externe 18 ainsi renforcée permet d'assurer un meilleur contrôle de la restriction stomacale lors du gonflage de la bande souple creuse 2 mise en place de manière stable autour de l'estomac.

La bande souple creuse 2 est réalisée par moulage de manière monobloc avec les deux matériaux constitutifs M1 et M2, M1 pour la paroi externe 18 comportant, dans la partie rectiligne distale de la bande souple creuse, au moins deux passants, et, M2 pour la paroi interne 17 mais il peut également y avoir une troisième matière M3 correspondant aux bandes longitudinales radio-opaques 21 parallèles ainsi qu'à la bande transversale radio-opaque 24.

### Le tube souple 5

L'extrémité distale 6^{d} du tube souple 5 est reliée à la bande souple 2 creuse par l'intermédiaire de l'ouverture 4 de gonflage ménagée dans la paroi externe 18.

En outre, le tube souple 5 est équipé, dans sa partie 16 d'extrémité distale 6^{d} rectiligne, de moyens de fermeture en boucle, par noeud coulant, qui sont complémentaires à ceux 11₁,11₂ de la partie 15 rectiligne de l'extrémité distale 9^{d} de la bande souple creuse 2. Les moyens de fermeture 10₁,10₂ sont des crans en forme de queues d'aronde dont les bases 10_{1b} et 10_{2b} sont de largeurs l₁, l₂ différentes. La largeur l₁ du cran 10₁ le plus proche de l'extrémité distale 6^{d} étant inférieure à la largeur l₂ de l'autre cran 10₂.

Selon une variante, ces largeurs l₁, l₂ peuvent être égales.

Une fois le tube souple 5 engagé au travers et au-delà des passants 11₁,11₂, les bases 10_{1b} et 10_{2b} des queues d'aronde 10₁ et 10₂ sont en butée anti-retour contre les arceaux 11₁,11₂, de manière à verrouiller la bande souple creuse 2 mise sous forme d'anneau en position fermée, comme montré sur la figure 8.

Pour réduire encore la force nécessaire au passage du tube souple 5, il peut également être envisagé de recouvrir sa surface extérieure d'un revêtement à faible coefficient de friction.

L'extrémité libre proximale 7^{p} du tube souple 5 peut être avantageusement obturée par un bouchon, de forme conique, qui évite l'introduction de matières dans le canal du cathéter et facilite l'introduction du cathéter dans les passants 11₁,11₂ lors de la fermeture de la bande souple creuse 2 pour former l'anneau. Après formation de l'anneau, le chirurgien ôte le bouchon et relie le tube souple aux moyens 8 de gonflage.

### Les moyens 8 de gonflage :

Les moyens 8 de gonflage sont constitués comme le montre la figure 8 d'une chambre 80 à cathéter implantable, pourvue d'un septum 81 au travers duquel une aiguille peut être plantée pour ponctionner et injecter du fluide de gonflage.

L'implant selon l'invention peut être mis en place par la voie coelioscopique. Pour aider le chirurgien, des moyens de repérage optique sont prévus sur la bande souple creuse 2 et/ou sur le tube souple 5.

### Variante de réalisation avec insert radio-opaque :

Suivant cette variante, la bande anneau 1 selon la mention comprend un insert radio-opaque 40 logé dans la bande souple 2 creuse, ledit insert 40 comportant de préférence un prolongement 41 d'au-moins l'une des parois 17-18-19-90-130 de la bande souple 2 creuse, est plus préférentiellement encore, un prolongement 41 de la paroi terminale distale 42 de la bande 2 creuse.

Suivant une forme particulièrement préférée de cette variante, la bande anneau 1 est obtenue à partir d'une pièce moulée intermédiaire 1', qui est démoulée par une ouverture 91 prévue en lieu et place de la paroi terminale distale 42 de la bande souple 2 creuse, ladite paroi terminale distale 42 étant fixée sur la pièce moulée intermédiaire 1' après démoulage et la paroi terminale distale 42 formant, de préférence, l'une des extrémités d'un insert radio-opaque 40 logé dans la bande souple 2 creuse.

Dans cette forme préférée de la variante, l'insert radio-opaque 40 est constitué d'une base 42 présentant un prolongement 41, comme montré sur les figures 11A-11B-12A-12B. La base 42 de l'insert 40 peut être, par exemple, réalisée en élastomère silicone, tandis que le prolongement 41 s'étendant à partir de cette base 42 et formant la partie radio-opaque proprement dite est, quant à lui, réalisé à partir d'un élastomère silicone chargé en un matériau radio-opaque, par exemple le sulfate de Baryum.

Comme cela apparaît plus nettement sur la figure 11A, le prolongement 41 est, par exemple, constitué par deux tiges parallèles 43 solidaires de la base 42 à l'une de leurs extrémités et reliées l'une à l'autre, à l'extrémité libre 44 du prolongement 41. Une entretoise 45 relie l'une à l'autre les tiges 43 du prolongement 41. Cet insert radio-opaque 40 montré aux figures 11A-11B, peut être introduit à l'intérieur de la bande souple 2 creuse de la bande anneau 1, par une ouverture 91 de démoulage prévue sur une pièce moulée intermédiaire 1', précurseur de la bande anneau 1 finie.

Une fois introduite à l'intérieur de la bande souple 2 creuse dans la chambre 30, le prolongement 42 est indépendant de la bande 2 creuse, tandis que la base 42 de l'insert 40 forme un bouchon venant obturer l'ouverture 91, pour former la paroi terminale distale 90 de la bande souple 2 creuse.

La fixation de la base 42 (ou bouchon) à la bande souple creuse, peut se faire par tout moyen approprié par exemple collage, soudage....

Le prolongement 43 a avantageusement une longueur inférieure à celle de la chambre 30 de la bande souple 2 creuse, de manière à ne pas obstruer l'ouverture 4 de gonflage.

Une fois la chambre creuse 30 remplie de fluide de gonflage, le prolongement 43 de l'insert 40 est immergé dans le fluide remplissant cette chambre 30 de la bande souple 2 creuse.

## Revendications

1. Bande-anneau (1) gastrique gonflable, ajustable destinée à être mise en place autour d'un segment du tube digestif pour le rétrécir, ladite bande-anneau comprenant :
▪ une bande (2) souple creuse, élastique, comprenant une paroi interne (17) et une paroi externe (18) définissant une chambre étanche gonflable (30), présentant une ouverture (4) de gonflage;
▪ un tube (5) souple de gonflage dont l'extrémité distale (6 ^{d}) est reliée à l'ouverture (4) de gonflage de la bande (2) et dont l'extrémité proximale (7 ^{p}) est destinée à être connectée à des moyens de gonflage (8);
▪ le tube (5) et la bande (2) étant équipés, dans leurs zones (6) et (9) d'extrémité distales respectives de moyens de fermeture (10) et (11) complémentaires aptes à coopérer entre eux pour assurer le verrouillage en position fermée d'un anneau (12) formé par rapprochement, selon le principe du noeud coulant, de l'extrémité distale (9^{d}) et de l'extrémité proximale (13^{p}) de la bande (2), notamment autour du segment de tube digestif à rétrécir;
les moyens de fermeture (10) du tube (5) étant au moins un cran et les moyens de fermeture (11) de la bande (2) étant au moins un passant, de préférence au moins deux passants, de sorte que l'extrémité proximale (7^{p}) du tube (5) est destinée à être engagée au travers dudit au moins un passant (11) de la bande (2) jusqu'à une position de fermeture verrouillée une fois que ledit au moins un cran (10) est amené au-delà dudit au moins un passant (11) ;
**caractérisée en ce que**
- la bande (2) souple creuse présente, au repos, sans engagement du tube (5) et de la bande (2) l'un dans l'autre pour former l'anneau (12), une partie préformée courbe (14) s'étendant à partir de sa zone (13) d'extrémité proximale et se prolongeant par une partie sensiblement rectiligne (15) jusqu'à l'extrémité libre distale (9^{d}) de la bande (2), ladite partie sensiblement rectiligne (15) présentant une longueur comprise entre 10 et 40 %, plus préférentiellement entre 17 et 35%, plus préférentiellement encore entre 21 et 33% d'une longueur totale de la bande souple creuse de manière à faciliter l'engagement du l'extrémité proximale (7^{p}) du tube (5) au travers dudit au moins un passant (11) de la bande (2), et
- ledit au moins un passant étant disposé sur la paroi externe (18) de la partie sensiblement rectiligne (15) de la bande souple creuse (2).

2. Bande-anneau (1) selon la revendication 1, **caractérisée en ce que** l'axe de la partie sensiblement rectiligne (15) de la bande (2) est sécant à la zone (6) d'extrémité distale du tube (5).

3. Bande-anneau (1) selon la revendication 1 ou 2, **caractérisée en ce que** la zone (6) d'extrémité distale du tube (5) présente, au repos et sans engagement du tube (5) et de la bande (2) l'un dans l'autre pour former l'anneau (12), une partie sensiblement rectiligne (16).

4. Bande-anneau selon la revendication 3, **caractérisée en ce que** l'angle A que forme la partie sensiblement rectiligne (15) distale de la bande (2) et la partie sensiblement rectiligne (16) distale du tube (15) est défini comme suit en °, selon un ordre croissant de préférence:
▪ A ≤ 60
▪ A ≤ 50
▪ 20 ≤ A ≤ 50
▪ 40 ≤ A ≤ 90
▪ A = 45.

5. Bande-anneau (1) selon l'une au moins des revendications précédentes, **caractérisée en ce que** la zone (13) d'extrémité proximale de la bande (2) est sensiblement rectiligne.

6. Bande-anneau (1) selon l'une au moins des revendications précédentes, **caractérisée en ce que** la bande (2) creuse est monobloc et **en ce qu'**elle est réalisée à partir d'au moins deux matières M1, M2, de préférence choisies parmi les élastomères silicone, se différenciant au moins par leurs duretés respectives D1, D2 avec D1 > D2, la matière M1 constituant au moins en partie la paroi externe (18) et la matière M2 constituant au moins en partie la paroi interne (17).

7. Bande-anneau (1) selon l'une au moins des revendications précédentes, **caractérisée en ce que** la paroi externe (18) de la bande souple creuse (2) comprend des arêtes longitudinales (22) et **en ce qu'**au moins les arêtes longitudinales (22) de la paroi externe (18) sont protubérantes et sont, de préférence, au moins en partie radio-opaque, notamment aux rayons X, ces arêtes longitudinales (22) comportant de préférence chacune une bande radio-opaque (21).

8. Bande-anneau (1) selon l'une au moins des revendications précédentes, **caractérisée en ce que** l'ouverture (4) de gonflage est ménagée dans la paroi externe de la zone (13^{p}) d'extrémité proximale de la bande (2) et **en ce que** cette paroi externe présente, sur sa face interne, des évidements, de préférence des rainures, plus préférentiellement encore des rainures longitudinales parallèles dont au moins une partie est disposée de part et d'autre de l'ouverture (4) de gonflage, ces évidements étant destinés à empêcher une occlusion de l'ouverture (4) de gonflage par la paroi interne de la bande (2) creuse en regard de cette ouverture (4), notamment lors du dégonflage.

9. Bande-anneau (1) selon l'une au moins des revendications précédentes, caractérisée qu'elle comprend un insert radio-opaque (40) logé dans la bande (2) creuse, ledit insert (40) comportant, de préférence, un prolongement (41) d'au moins l'une des parois (17,18,19,90,130) de la bande (2) creuse, et plus préférentiellement encore, un prolongement (41) de la paroi terminale distale (90) de la bande (2) creuse.

10. Bande-anneau (1) selon l'une quelconque des revendications 1 à 9, **caractérisée en ce qu'**elle est obtenue par multi-injection séquentielle au sein d'un même moule.

11. Procédé d'obtention de la bande-anneau selon l'une au moins des revendications précédentes, **caractérisé en ce qu'**il consiste essentiellement à réaliser un moulage, de préférence monobloc par multi-injection séquentielle au sein d'un même moule.

12. Nécessaire pour la réalisation par voie chirurgicale d'un rétrécissement d'un segment du tube digestif, de préférence de l'estomac, comprenant:
* une bande-anneau (1) selon l'une au moins des revendications 1 à 10;
* et un module de gonflage équipé d'un septum et destiné à être relié à l'extrémité proximale (7^{p}) du tube (5) souple, pour permettre l'injection ou l'extraction in situ d'un fluide de gonflage pour le gonflage ou le dégonflage de la bande (2) creuse.

## Patentansprüche

1. Aufblasbares, anpassbares Magen-Ringband (1), welches dazu vorgesehen ist, um einen Abschnitt des Verdauungstraks herum platziert zu werden, um ihn verengen, wobei das Ringband umfasst:
- ein flexibles, hohles, elastisches Band (2), umfassend eine innere Wand (17) und eine äußere Wand (18), welche eine dichte aufblasbare Kammer (30) definieren, welche eine Öffnung (4) zum Aufblasen aufweist;
- eine flexible Aufblasröhre, deren distales Ende (6^{d}) an der Öffnung (4) zum Aufblasen des Bands (2) angeschlossen ist, und deren proximales Ende (7^{p}) dazu vorgesehen ist, mit Aufblasmitteln (8) verbunden zu werden;
- wobei die Röhre (5) und das Band (2) in ihren jeweiligen Zonen (6) und (9) der distalen Enden mit komplementären Schließmitteln (10) und (11) ausgestattet sind, welche in der Lage sind, miteinander zusammenzuwirken, um das Festsetzen eines Rings (12) in geschlossener Position sicherzustellen, welcher durch eine Annäherung des distalen Endes (9^{d}) und des proximalen Endes (13^{p}) des Bands (2) nach dem Schlingenprinzip gebildet ist, insbesondere um den zu verengenden Abschnitt des Verdauungstrakts herum;
wobei die Schließmittel (10) der Röhre (5) wenigstens einen Einschnitt aufweisen und die Schließmittel (11) des Bands (2) wenigstens eine Schlaufe aufweisen, vorzugsweise wenigstens zwei Schlaufen, derart, dass das proximale Ende (7^{p}) der Röhre (5) dazu vorgesehen ist, durch die wenigstens eine Schlaufe (11) des Bands (2) bis zu einer festgesetzten Schließposition einzugreifen, sobald der wenigstens eine Einschnitt (10) über die wenigstens eine Schlaufe (11) hinaus geführt wird;
**dadurch gekennzeichnet, dass**
- das flexible, hohle Band (2) in Ruhe, ohne Eingriff der Röhre (5) und des Bands (2) miteinander zum Bilden des Rings (12) einen vorgeformten Kurvenabschnitt (14) aufweist, welcher sich ausgehend von seiner Zone (13) des proximalen Endes erstreckt und in einem im Wesentlichen geradlinigen Abschnitt (15) bis zu dem freien distalen Ende (9^{d}) des Bands (2) weiter verläuft, wobei der im Wesentlichen geradlinige Abschnitt (15) eine Länge aufweist, welche zwischen 10 und 40%, vorzugsweise zwischen 17 und 35%, weiter vorzugsweise zwischen 17 und 35%, weiter vorzugsweise zwischen 21 und 33% einer Gesamtlänge des flexiblen, hohlen Bands beträgt, um den Eingriff des proximalen Endes (7^{p}) der Röhre (5) durch die wenigstens eine Schlaufe (11) des Bands (2) hindurch zu erleichtern, und
- die wenigstens eine Schlaufe an der äußeren Wand (18) des im Wesentlichen geradlinigen Abschnitts (15) des flexiblen, hohlen Bands (2) angeordnet ist.

2. Ringband (1) nach Anspruch 1, **dadurch gekennzeichnet, dass** die Achse des im Wesentlichen geradlinigen Abschnitts (15) des Bands (2) die Zone (6) des distalen Endes der Röhre (5) schneidet.

3. Ringband (1) nach Anspruch 1 oder 2, **dadurch gekennzeichnet, dass** die Zone (6) des distalen Endes der Röhre (5) in Ruhe und ohne Eingriff der Röhre (5) und des Bands (2) miteinander zum Bilden des Rings (12) einen im Wesentlichen geradlinigen Abschnitt (16) bildet.

4. Ringband nach Anspruch 3, **dadurch gekennzeichnet, dass** der Winkel A, welchen der im Wesentlichen geradlinige distale Abschnitt (15) des Bands (2) und der im Wesentlichen geradlinige distale Abschnitt (16) der Röhre (15) bilden, wie folgt in ° definiert ist, nach aufsteigender Reihenfolge der Bevorzugung:
- A ≤ 60
- A ≤ 50
- 20 ≤ A ≤50
- 40 ≤ A ≤90
- A=45

5. Ringband (1) nach wenigstens einem der vorhergehenden Ansprüche, **dadurch gekennzeichnet, dass** die Zone (13) des proximalen Endes des Bands (2) im Wesentlichen geradlinig ist.

6. Ringband (1) nach wenigstens einem der vorhergehenden Ansprüche, **dadurch gekennzeichnet, dass** das hohle Band einstückig ist, und dass es ausgehend von wenigstens zwei Materialien M1, M2 gebildet ist, welche vorzugsweise aus Silikon-Elastomeren ausgewählt sind, welche sich wenigstens durch jeweilige Härte D1, D2 unterscheiden, wobei D1 > D2, wobei das Material M1 wenigstens teilweise die äußere Wand (18) bildet und das Material M2 wenigstens teilweise die innere Wand (17) bildet.

7. Ringband (1) nach wenigstens einem der vorhergehenden Ansprüche, **dadurch gekennzeichnet, dass** die äußere Wand (18) des flexiblen, hohlen Bands (2) longitudinale Grate (22) umfasst, und dass wenigstens die longitudinalen Grate (22) der äußeren Wand (18) hervorstehend sind und vorzugsweise wenigstens teilweise strahlenundurchlässig sind, nämlich für Röntgen-Strahlen, wobei die longitudinalen Grate (22) vorzugsweise jeweils ein strahlenundurchlässiges Band (21) umfassen.

8. Ringband (1) nach wenigstens einem der vorhergehenden Ansprüche, **dadurch gekennzeichnet, dass** die Öffnung (4) zum Aufblasen in der äußeren Wand der Zone (13^{p}) des proximalen Endes des Bands (2) aufgenommen ist, und dass diese äußere Wand an ihrer inneren Fläche Aussparungen, vorzugsweise Rillen, weiter vorzugsweise longitudinale parallele Rillen, aufweist, von welchen wenigstens ein Teil beiderseits der Öffnung (4) zum Aufblasen angeordnet ist, wobei diese Aussparungen dazu vorgesehen sind, ein Verschließen der Öffnung (4) zum Aufblasen durch die innere Wand des hohlen Bands (2) bezüglich dieser Öffnung (4) zu verhindern, insbesondere während des Ablassens.

9. Ringband (1) nach wenigstens einem der vorhergehenden Ansprüche, **dadurch gekennzeichnet, dass** es einen strahlenundurchlässigen Einsatz (40) umfasst, welcher in dem hohlen Band (2) aufgenommen ist, wobei der Einsatz (40) vorzugsweise einen Fortsatz (41) von wenigstens einer der Wände (17, 18, 19, 90, 130) des hohlen Bands (2) umfasst, und weiter vorzugsweise einen Fortsatz (41) der distalen Endwand (90) des hohlen Bands (2).

10. Ringband (1) nach einem der Ansprüche 1 bis 9, **dadurch gekennzeichnet, dass** es durch aufeinander folgendes mehrfaches Spritzgießen in einer gleichen Form hergestellt ist.

11. Verfahren zum Herstellen des Ringbands nach wenigstens einem der vorhergehenden Ansprüche, **dadurch gekennzeichnet, dass** es im Wesentlichen aus einem Erhalten eines Gusses besteht, vorzugsweise einstückig, durch aufeinander folgendes mehrfaches Spritzgießen in einer gleichen Form.

12. Ausrüstung zum Herstellen einer Verengung eines Abschnitts des Verdauungstrakts, vorzugsweise des Magens, auf chirurgischem Weg, umfassend:
- ein Ringband (1) nach wenigstens einem der Ansprüche 1 bis 10;
- und ein Modul zum Aufblasen, welches mit einem Septum ausgestattet und dazu vorgesehen ist, an ein proximales Ende (7^{p}) der hohlen Röhre (5) angeschlossen zu werden, um das in situ Eingeben oder Entnehmen eines Aufblasfluids zu erlauben, für das Aufblasen oder Ablassen des hohlen Bands (2).

## Claims

1. An inflatable and adjustable gastric band (1) to be positioned around a segment of the digestive tract in order to narrow the latter, said gastric band comprising:
▪ a hollow flexible band (2), which is resilient and comprises an inner wall (17) and an outer wall (18) defining an inflatable leaktight chamber (30) with an inflation opening (4);
▪ a flexible inflation tube (5) whose distal end (6^{d}) is connected to the inflation opening (4) of the band (2), and whose proximal end (7^{p}) is intended to be connected to inflation means (8);
▪ the tube (5) and the band (2) being provided, in the respective distal end areas (6) and (9) thereof, with complementary closure means (10) and (11) that are able to mutually engage so as to lock a ring (12) in a closed position, said ring being formed by bringing the distal end (9^{d}) and the proximal end (13^{p}) of the band (2) together according to the principle of a slip knot, in particular around the segment of the digestive tract to be narrowed;
the closure means (10) of the tube (5) being at least one notch and the closure means (11) of the band (2) being at least one loop, preferably two loops, such that the proximal end (7^{p}) of the tube (5) is intended to be engaged through said at least one loop (11) of the band (2) as far as a locked closure position once said at least one notch (10) is brought beyond said at least one loop (11);
**characterized in that**
- the hollow flexible band (2), when not in use and when the tube (5) and the band (2) are not engaged in each other to form the ring (12), has a preformed curved part (14) which extends starting from the proximal end area (13) thereof and which is continued via a substantially rectilinear part (15) to the free distal end (9^{d}) of the band (2), said substantially rectilinear part (15) having a length between 10 and 40%, more preferably between 17 and 35%, even more preferably between 21 ad 33% of a total length of the hollow flexible band so as to facilitate the engagement of the proximal end (7^{p}) of the tube (5) through said at least one loop (11) of the band (2), and
- said at least one loop being arranged on the outer wall (18) of the substantially rectilinear part (15) of the hollow flexible band (2).

2. The gastric band (1) as claimed in claim 1, **characterized in that** the axis of the substantially rectilinear part (15) of the band (2) intersects the distal end area (6) of the tube (5).

3. The gastric band (1) as claimed in claim 1 or 2, **characterized in that** the distal end area (6) of the tube (5) has a substantially rectilinear part (16) when not in use and when the tube (5) and the band (2) are not engaged in each other to form the ring (12).

4. The gastric band as claimed in claim 3, **characterized in that** the angle A formed by the substantially rectilinear distal part (15) of the band (2) and the substantially rectilinear distal part (16) of the tube (15) is defined as follows in °, according to an increasing order of preference:
▪ A ≤ 60
▪ A ≤ 50
▪ 20 ≤ A ≤ 50
▪ 40 ≤ A ≤ 90
▪ A = 45.

5. The gastric band (1) as claimed in at least one of the preceding claims, **characterized in that** the proximal end area (13) of the band (2) is substantially rectilinear.

6. The gastric band (1) as claimed in at least one of the preceding claims, **characterized in that** the hollow band (2) is in one piece, and **in that** it is produced from at least two materials M1, M2, which are preferably chosen from silicone elastomers and differ from each other at least in terms of their respective hardness D1, D2, with D1 > D2, the material M1 constituting at least part of the outer wall (18), and the material M2 constituting at least part of the inner wall (17).

7. The gastric band (1) as claimed in at least one of the preceding claims, **characterized in that** the outer wall (18) of the hollow flexible band (2) comprises longitudinal edges (22) and **in that** the longitudinal edges (22) of the outer wall (18) are protuberant and are preferably at least partially radiopaque, especially to X-rays, these longitudinal edges (22) each preferably having a radiopaque band (21).

8. The gastric band (1) as claimed in at least one of the preceding claims, **characterized in that** the inflation opening (4) is formed in the outer wall of the proximal end area (13^{p}) of the band (2), and **in that** this outer wall has, on its inner face, recesses, preferably grooves, still more preferably parallel longitudinal grooves, of which at least some are arranged on each side of the inflation opening (4), these recesses being intended to prevent occlusion of the inflation opening (4) by the inner wall of the hollow band (2) opposite this opening (4), especially during deflation.

9. The gastric band (1) as claimed in at least one of the preceding claims, **characterized in that** it comprises a radiopaque insert (40) lodged in the hollow band (2), said insert (40) preferably having a continuation (41) of at least one of the walls (17, 18, 19, 90, 130) of the hollow band (2), and still more preferably a continuation (41) of the distal end wall (90) of the hollow band (2).

10. The gastric band (1) as claimed in at least one of claims 1 to 9, **characterized in that** it is obtained by sequential multi-injection within one and the same mold.

11. A method for obtaining the gastric band as claimed in at least one of the preceding claims, **characterized in that** it principally involves producing a molding, preferably in one piece, by sequential multi-injection within one and the same mold.

12. A kit for surgically narrowing a segment of the digestive tract, preferably the stomach, said kit comprising:
* a gastric band (1) as claimed in at least one of claims 1 to 10;
* and an inflation module equipped with a septum and intended to be connected to the proximal end (7^{p}) of the flexible tube (5), in order to permit *in situ* injection or extraction of an inflation fluid for inflating or deflating the hollow band (2).
